# EUROPÄISCHE PATENTSCHRIFT

(11) **EP 1 089 763 B1**
(45) Veröffentlichungstag und Bekanntmachung des Hinweises auf die Patenterteilung: **25.09.2002**
(21) Anmeldenummer: 99939942.1
(22) Anmeldetag: 24.06.1999
(51) Int. Cl.: A61K 47/48, A61K 38/00, A61P 19/10

(54) **MITTEL ZUR SPEZIFISCHEN HEMMUNG OSTEOKLASTÄRER KNOCHENRESORPTION**
AGENTS FOR SPECIFICALLY INHIBITING OSTEOCLASTIC BONE RESORPTION
AGENTS POUR L'INHIBITION SPECIFIQUE DE LA RESORPTION OSTEOCLASTIQUE

(30) Priorität: 24.06.1998 DE 19828068
(43) Veröffentlichungstag der Anmeldung: 11.04.2001
(73) Patentinhaber: Dietsch, Peter, 14169 Berlin (DE); Halbach, Alexandra, 12205 Berlin (DE)
(72) Erfinder: Dietsch, Peter, 14169 Berlin (DE); Halbach, Alexandra, 12205 Berlin (DE)
(74) Vertreter: Baumbach, Friedrich, Dr.
(86) Internationale Anmeldenummer: DE9901839
(87) Internationale Veröffentlichungsnummer: WO99066910

(56) Entgegenhaltungen:
- EP-A- 0 201 057
- WO-A-93/20229
- WO-A-96/09067
- US-A- 5 354 773
- DAVID P ET AL: "THE VACUOLAR H+-ATPHASE: A POTENTIAL TARGET FOR DRUG DEVELOPMENT IN BONE DISEASES" EXPERT OPINION ON INVESTIGATIONAL DRUGS,GB,ASHLEY PUBLICATIONS LTD., LONDON, Bd. 4, Nr. 8, 1. Januar 1995 (1995-01-01), Seiten 725-740, XP000655119 ISSN: 1354-3784
- PIERCE, WILLIAM M., JR. ET AL: "Bone-targeted carbonic anhydrase inhibitors: effect of a proinhibitor on bone resorption in vitro" PROC. SOC. EXP. BIOL. MED. (1987), 186(1), 96-102 , XP000877284
- BLAIR H C (REPRINT) ET AL: "RECENT ADVANCES TOWARD UNDERSTANDING OSTEOCLAST PHYSIOLOGY" CLINICAL ORTHOPAEDICS AND RELATED RESEARCH, (SEP 1993) NO. 294, PP. 7-22. ISSN: 0009-921X., XP000884924 UNIV ALABAMA, DEPT PATHOL, 535 LYONS HARRISON BLDG, UAB STN, BIRMINGHAM, AL, 35294 (Reprint);DEPT VET AFFAIRS MED CTR, LAB SERV, BIRMINGHAM, AL, 00000; SHRINERS HOSP CRIPPLED CHILDREN, ST LOUIS UNIT, ST LOUIS, MO, 00000; WASHINGTON UNIV, SCH MED, DEPT CELL BIOL & PHYSIOL, ST LOUIS, MO, 63110; WASHIN
- KENNY A D: "Role of carbonic anhydrase in bone: plasma acetazolamide concentrations associated with inhibition of bone loss." PHARMACOLOGY, (1985) 31 (2) 97-107. , XP000884920
- BARON R: "Polarity and membrane transport in osteoclasts." CONNECTIVE TISSUE RESEARCH, (1989) 20 (1-4) 109-20. REF: 27 , XP000884892
- SASAKI, TAKAHISA (1) ET AL: "Expression of vacuolar H +- ATPase in osteoclasts and its role in resorption." CELL AND TISSUE RESEARCH, (1994) VOL. 278, NO. 2, PP. 265-271. , XP000884899
- BLAIR H C ET AL: "Receptor-mediated uptake of a mannose -6-phosphate bearing glycoprotein by isolated chicken osteoclasts." JOURNAL OF CELLULAR PHYSIOLOGY, (1988 DEC) 137 (3) 476-82. , XP000892354
- BARON: "polarized secretion of lysosomial enzymes:..." J. CELL BIOLOGY, Bd. 106, Nr. 6, 1988, Seiten 1863-11872, XP000892360
- BARON R: "Molecular mechanisms of bone resorption by the osteoclast." ANATOMICAL RECORD, (1989 JUN) 224 (2) 317-24. REF: 66 , XP000889769
- SAUER G R ET AL: "A facilitative role for carbonic anhydrase activity in matrix vesicle mineralization." BONE AND MINERAL, (1994 JUL) 26 (1) 69-79. , XP000884909
- HENTUNEN T A (REPRINT) ET AL: "INHIBITION OF BONE-RESORPTION BY A MONOCLONAL-ANTIBODY THAT REACTS WITH A 150-KD MEMBRANE-PROTEIN IN CHICKEN OSTEOCLASTS" JOURNAL OF BONE AND MINERAL RESEARCH, (1991) VOL. 6, NO. 10, PP. 1091-1097., XP000892353 UNIV OULU, DEPT ANAT, KAJAANINTIE 52A, SF-90220 OULU, FINLAND (Reprint)

## Beschreibung

Die Erfindung betrifft Mittel zur spezifischen Hemmung osteoklastärer Knochenresorption. Anwendungsgebiete der Erfindung sind die Medizin und die pharmazeutische Industrie.

Knochen unterliegt dauerndem Umbau (remodeling), der für die Anpassung an geänderte Belastung, Reparaturprozesse und für die Funktion des Knochens als Calciumspeicher Voraussetzung ist. Im gesunden Knochen sind Anbau durch Osteoblasten und Abbau durch Osteoklasten miteinander gekoppelt und fein reguliert. Die Säureproduktion durch Osteoklasten dient der Überfuhrung von unlöslichem, tertiärem Calziumphosphat (Apatit), das mehr als 90% des Knochenminerals ausmacht, in lösliches, primäres Calciumphosphat. Enzyme, die von Osteoklasten in die Resorptionslakune sezerniert werden und die organische Matrix abbauen, haben ihr pH-Optimum im Sauren. Die Säureproduktion ist damit auch Voraussetzung für deren Aktivität.

Dieses Gleichgewicht ist bei Knochenerkrankungen gestört. Überwiegt der Abbau, so verliert der Knochen an Festigkeit, es entstehen Mikrofrakturen im spongiösen Knochen - typisches Beispiel hierfür ist das (langsame) Zusammenbrechen von Wirbelkörpern - oder es kommt zu Frakturen unter Belastung. Typisch hierfür sind Brüche des Schenkelhalses, der Unterarme und Handwurzelknochen.
In der Bundesrepublik Deutschland leben 3 Millionen Menschen mit Wirbelkörperfrakturen. 70000 Oberschenkelhalsbrüche sind jährlich festzustellen. Die Folgekosten der Oberschenkelhalsfrakturen bewegen sich zwischen 2,5 - 5 Milliarden DM pro Jahr.
Je nach ihrer Äthiologie werden verschiedene Erkrankungen unterschieden, die mit einer Minderung der Knochenmasse einhergehen.
Zahlenmäßig am bedeutsamsten sind die postmenopausale Osteoporose der Frau und in zunehmenden Maße die Altersosteoporose, die beide Geschlechter betrifft.
Daneben gibt es stoffwechselbedingte Verluste der Knochenmasse beim Hyperparathyreoidismus, Nierenfunktionsstörungen (renale Osteopathie) und Vitamin D-Mangel (Osteomalazie). Erhöhte Osteoklastenaktivität findet man beim Morbus Paget. Knochentumoren bzw. Metastasen im Knochen (z.B. Mammakarzinom) gehen häufig mit erhöhter Osteoklastenaktivität einher. Selbst bei Arthritis kann eine Aktivierung von Osteoklasten zu gesteigerter Knochenresorption führen.

Derzeitige Therapiekonzepte beinhalten im Falle der Osteoporose, die den ganz überwiegenden Teil der Knochenerkrankungen ausmacht, ausreichende Calcium- und Vitamin D₃ - Zufuhr, die ergänzt wird durch Fluorid, das die Bildung neuen Knochenminerals (allerdings mechanisch wenig stabil) anregt.

Bei der postmenopausalen Osteoporose bietet sich die Behandlung mit Östrogenen an, die bei den betroffenen Patientinnen allerdings wenig Akzeptanz findet.
Die mit hohen Erwartungen begleitete Einführung synthetischen Calcitonins, des Hormons, das Osteoklasten hemmt, ist zum Teil von den beteiligten Firmen rückgängig gemacht worden. Es findet seinen Hauptanwendung nur mehr bei der schmerzhaft foudroyanten Osteoporose infolge schmerzlindernder Wirkung. Die Anwendung ist zeitlich begrenzt.

Bisphosphonate haben sich bei vielen Knochenerkrankungen bewährt, neuere Entwicklungen haben Nebenwirkungen verringert. Bisher sind nur wenige für die Behandlung der Osteoporose zugelassen. Ihr Wirkungsmechanismus ist jedoch trotz jahrzehntelanger Forschung völlig ungeklärt.

Als einzige Knochenzellen besitzen Osteoklasten das Enzym Carboanhydrase, das die reversible Hydratisierung von CO₂ zu Kohlensäure katalysiert. Die durch spontane Dissoziation der Kohlensäure entstehenden H⁺ - Ionen werden mit Hilfe einer vesikulären H⁺-ATPase in die Resorptionslakune unterhalb des aktiven Osteoklasten gepumpt und bewirken hier die Auflösung des Knochenminerals.
Bereits 1960 konnten Siegmund et al. zeigen, daß Inhibitoren der Carboanhydrase die Calciumkonzentration im Serum von Hühnern erniedrigen und die gegengerichtete Wirkung von Parathyrin blockieren.
Zwischen 1950 und 1960 gab es eine umfangreiche Literatur, die die Verwendung von Carboanhydraseinhibitoren bei verschiedenen Erkrankungen beschrieb; nicht jedoch solchen des Knochens. Bis auf die zeitweise Bedeutung des Diamox (Azetazolamid) zur Behandlung des Glaukoms, hat keine Eingang in die Praxis gefunden.
Eine systemische Anwendung von Inhibitoren der Carboanhydrase verbietet sich deshalb, weil das Enzym in vielen Organen vorkommt, in denen es wichtige Funktionen ausübt, wie zum Beispiel beim CO₂ Transport (Erythrozyten, Lunge), bei der Säureproduktion (Magen, Niere, Knochen), der Bicarbonatproduktion (Pankreas), zur Pufferung (Blut) u.a.,
Ein genetisch bedingter Mangel - vergleichbar einer längerfristigen Anwendung von Inhibitoren des Isoenzyms Carboanhydrase II - führt zum Krankheitsbild der Osteopetrose, das begleitet ist von Nierenschäden und Basalganglienverkalkung.

Der Erfindung liegt die Aufgabe zugrunde, ein Mittel zur Verfügung zu stellen, mit dem die Osteoklasten spezifisch inhibiert werden können, wodurch keine Säurebildung und dadurch verursachte Auflösung des Knochenminerals mehr erfolgen kann.

Osteoklasten und Monozyten-Makrophagen entstammen einer gemeinsamen Vorläuferzelle, der multipotenten, hämatopoetischen Stammzelle. Obwohl sie sich nicht mehr ineinander umwandeln lassen, besitzen Osteoklasten und Makrophagen die Fähigkeit zur Phagozytose (Endozytose). Voraussetzung hierfür ist die Bindung der zu phagozytierenden Moleküle (oder Partikel) an die Zelloberfläche mit Hilfe spezieller Rezeptoren.

Es wurde gefunden, daß auf den Osteoklasten Mannose - Rezeptoren vorhanden sind, die eine spezifische Eigenschaft dieser Zellen darstellen. Sie finden sich zwar auch auf Vorläuferzellen der Osteoklasten und auf Makrophagen, das Enzym Carboanhydrase wird aber ausschließlich in Osteoklasten exprimiert. Dieser wichtige Befund ist Ausgangspunkt der Erfindung.

Die Erfindung wird gemäß den Ansprüchen realisiert.

Das erfindungsgemäBe Mittel ist dadurch gekennzeichnet, daß es einen Inhibitor enthält, der spezifisch an Osteoklasten bindet und deren säurebildende bzw. -transportierende Enzyme hemmt. Die Varianten der Erfindung bestehen darin, daß entweder die Carboanhydrase oder die säuretransportierende H⁺⁻ATPase gehemmt werden.

Das erfindungsgemäße Mittel besteht aus einem Stoff, der spezifisch an die Rezeptoren der Osteoklasten bindet und einem daran gebundenen Wirkstoff. Der an die Rezeptoren der Osteoklasten bindende Stoff ist bevorzugt Mannose, kann aber auch Mannan sein. Der Wirkstoff ist bevorzugt ein Sulfonamid, besonders bevorzugt ein aromatisches oder heteroaromatisches Sulfonamid. In Betracht kommende Verbindungen sind Methazolamid, Azetazolamid, Ethoxzolamid, Chlorzolamid, p-Aminomethylbenzolsulfonamid (Marfanil), p-Aminobenzolsulfonamid (Sulfanilamid) und 2.4-Diaminoazobenzol-4'-sulfonamid (Prontosil).

Hemmstoffe der H⁺⁻ATPase sind u. a. Omeprazol, Bafilomycin A1 und Concanavalin.

Zum Umfang der Erfindung gehört auch die Verwendung des neuen Mittels. Es wird vorteilhaft zur Behandlung von Osteoporose und weiterer analoger Knochenerkrankungen eingesetzt.

Der für Osteoklasten spezifische Carboanhydrase-Inhibitor bindet speziell an diese Zellen und wird aufgenommen, er gelangt aber nicht in andere Carboanhydrase-haltige Zellen. Beides wird erfindungsgemäß durch Kopplung aromatischer Sulfonamide mit einem oder mehreren Mannose-Molekülen erreicht.
a) spezifische Bindung an den Mannose-Rezeptor der Osteoklasten und Aufnahme in die Zelle durch Endozytose.
b) b) durch die hydrophile Eigenschaft der Mannose, die den direkten Durchgang durch die hydrophobe Zellmembran und dadurch die Penetration in andere Zellen verhindert.

Die Aufnahme eines solchen Inhibitors durch Vorläuferzellen der Osteoklasten stellt keinen Nachteil, sondern einen weiteren Vorteil dar, da er hier keine Wirkung besitzt - da keine Carboanhydrase vorhanden - aber als Depot wirkt.
Eine Medikamentation kann damit in Abständen von einigen Wochen erfolgen.

Die praktische Durchführbarkeit des beschriebenen Mittels und Verwendungsverfahrens wird nachfolgend durch Synthese einer Modellverbindung und deren Prüfung an isolierten Osteoklasten sowie an einem Tiermodell nachgewiesen.

### Ausführungsbeispiele

### Synthese von N-α-D-Mannopyranosylphenyl-N'-p-amidosulfonylbenzylthioharnstoff (1)

Isothiocyanate reagieren mit primären Aminogruppen zu stabilen Derivaten des Thioharnstoffs.

### Formel:

N-α-D-Mannopyranosylphenyl-N'-amidosulfonyl-p-benzylthioharnstoff wird folgendermaßen synthetisiert: 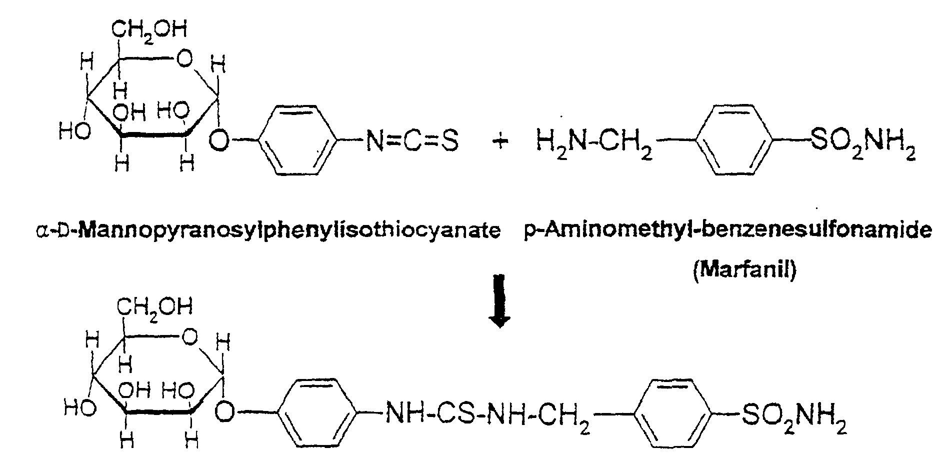 2,5 ml einer 16 mmol/l Lösung von p-Aminomethylbenzolsulfonamid-HCl in 20 mmol/l Tris-Puffer, pH 9,0, werden mit 2,5 ml einer 16 mmol/l Lösung von α-D-Mannopyranosylphenylisothiocyanat in 20 mmol/l Tris-Puffer, pH 9,0 versetzt und bei Raumtemperatur 30 Stunden lang gerührt, wobei der pH-Wert auf 7 sinkt.

Die Lösung wird 24 Stunden bei 3°C aufbewahrt. Der gebildete Niederschlag wird abzentrifugiert und dreimal an der Zentrifuge mit Methanol gewaschen. Läßt man den Überstand für einen weiteren Tag bei 4°C stehen, bildet sich erneut ein Niederschlag, der wie oben behandelt und mit dem ersten vereinigt wird.

Nach Trocknen im Exsikkator über Kieselgel beträgt die Ausbeute 92 %. **1** schmilzt bei 220°C unter Zersetzung. Als Nachweis für die erfolgreiche Kopplung und die Reinheit dient eine Dünnschichtchromotographie (Chloroform/Methanol/Wasser 65:35:8 als Laufmittel). Es zeigt sich ein einzelner Substanzfleck, dessen Laufstrecke nicht mit denen der Ausgangsverbindungen identisch ist.

Die zusätzlich eingeführte Gruppe hat keinen Einfluß auf die Fähigkeit des Sulfonamid zur Hemmung von Carboanhydrase.
Die Inhibitorkonstante von **1** für das in Osteoklasten exprimierte Isoenzym Carboanhydrase II beträgt K_{I} = 0,8 µmol/l. Sie entspricht damit der für p-Aminomethylbenzolsulfonamid zu K_{I} = 1µmol/l bestimmten.

Ein weiterer für Osteoklasten spezifischer Inhibitor der Carboanhydrase läßt sich ausgehend von Acetazolamid (Diamox), das ein zur Behandlung des Glaukoms zugelassenes Arzneimittel ist, auf folgende Weise synthetisieren:

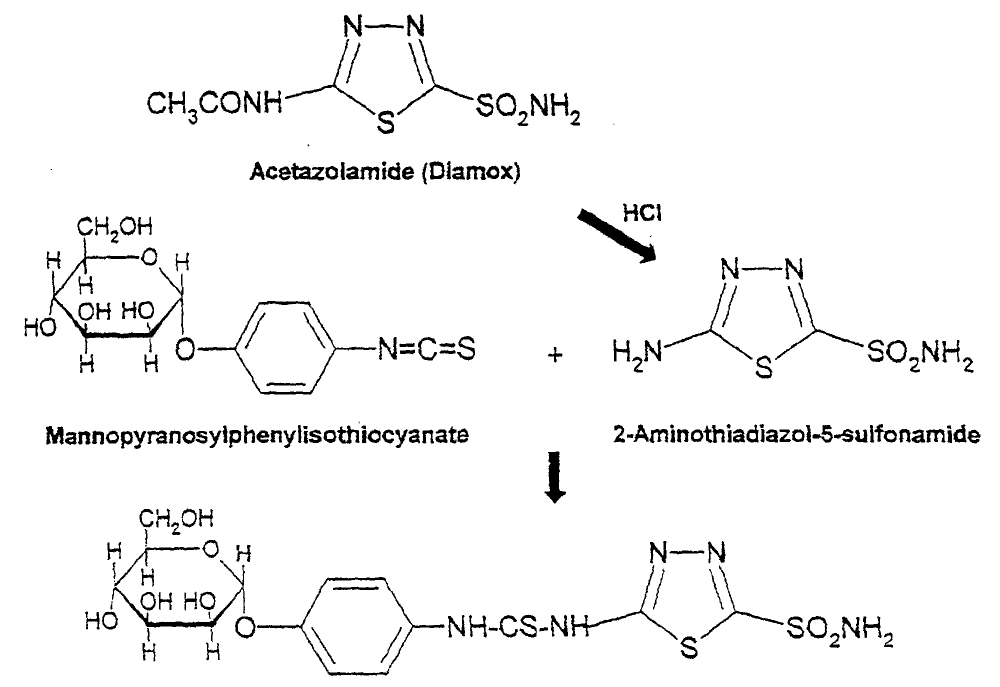

### N-α-D-Mannopyranosylphenyl-N'-5-amidosulfonyl-1,3,4-thiadiazolyl-(2)-thioharnstoff

2-Acetylamino-1,3,4-thiadiazol-5-sulfonamid wird durch Kochen am Rückfluß mit 0,1 mol/l HCI deacetyliert, wobei es in Lösung geht. Nach Abkühlen wird die Lösung mit Natriumcarbonat auf pH 5 gebracht und über Nacht im Kühlschrank aufbewahrt. Die entstandenen Kristalle werden abgesaugt, mit wenig kaltem Wasser gewaschen und aus Wasser umkrisallisiert.
Das entstandene 2-Amino-1,3,4-thiadiazol-5-sulfonamid wird in der oben beschriebenen Weise mit Mannopyranosylphenylisothiocyanat zum Thioharnstoff umgesetzt, wobei vorteilhaft die Lösungen zuvor mit Stickstoff durchgast werden, um Nebenreaktionen zu vermeiden.

Diese Verbindung besitzt eine tausendfach kleinere Inhibitorkonstante als die zuvor beschriebene. Die Hemmung des Enzyms und damit der Knochenresorption läßt sich damit durch geringe Konzentrationen erreichen.

### Nachweis der Aufnahme von 1 in Osteoklasten:

Carboanhydrase II und 5-Dimethylamino-naphthalin-1-sulfonamid (DNSA) bilden eine hellblau fluoreszierende Verbindung mit der Dissoziationskonstante K_{D} =2,4 x 10⁻⁷ mol/l. DNSA kann Zellmembranen passieren. Das Maximum der Anregung der Fluoreszenz liegt bei einer Wellenlänge von 320 nm, das Maximum der Emission bei 470 nm. Diese Fluoreszenz läßt sich gut von der des ungebundenen DNSA unterscheiden, da diese grün und sehr viel schwächer ist. Sie hat ihr Emissionsmaximum bei 580 nm. Die Carboanhydrase bindet DNSA im stöchiometrischen Verhältnis 1:1. Für **1** wurden die Konzentrationsverhältnisse so gewählt, daß es mit DNSA um die Bindung an die Carboanhydrase konkurrieren kann, was durch die Abnahme der Fluoreszenz deutlich wird.

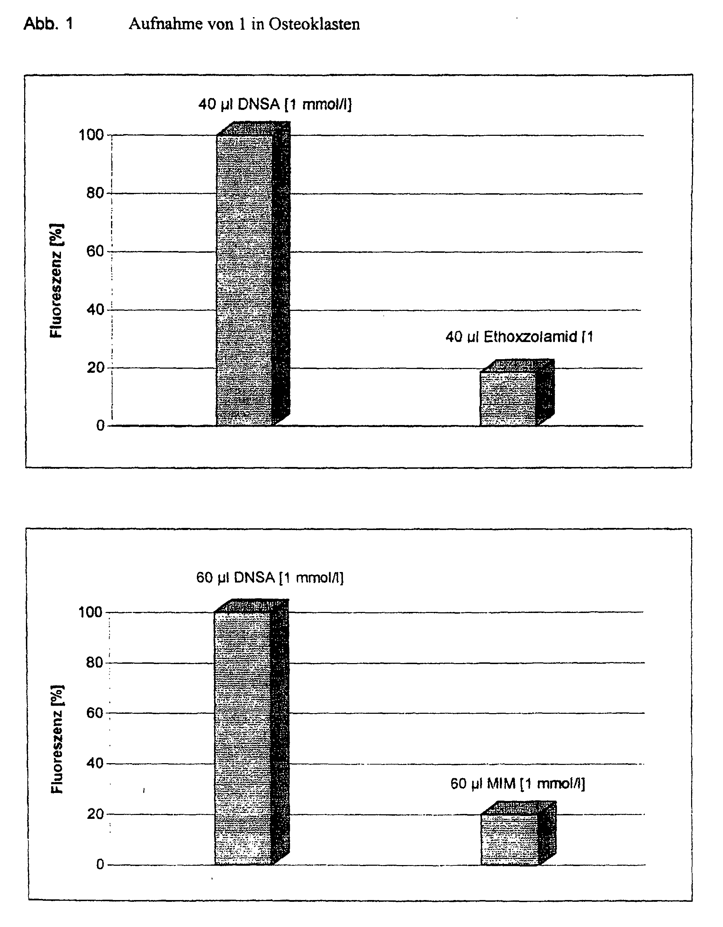

Wie der Abb. 1 entnommen werden kann, ist Ethoxzolamid (6-Ethoxybenzothiaziol-2-sulfonamid) membrangängig und hat eine kleinere Inhibitorkonstante für die Carboanhydrase (2,5 x 10⁻¹⁰ mol/l) und kann somit DNSA aus dem DNSA-Carboanhydrase-Komplex verdrängen.

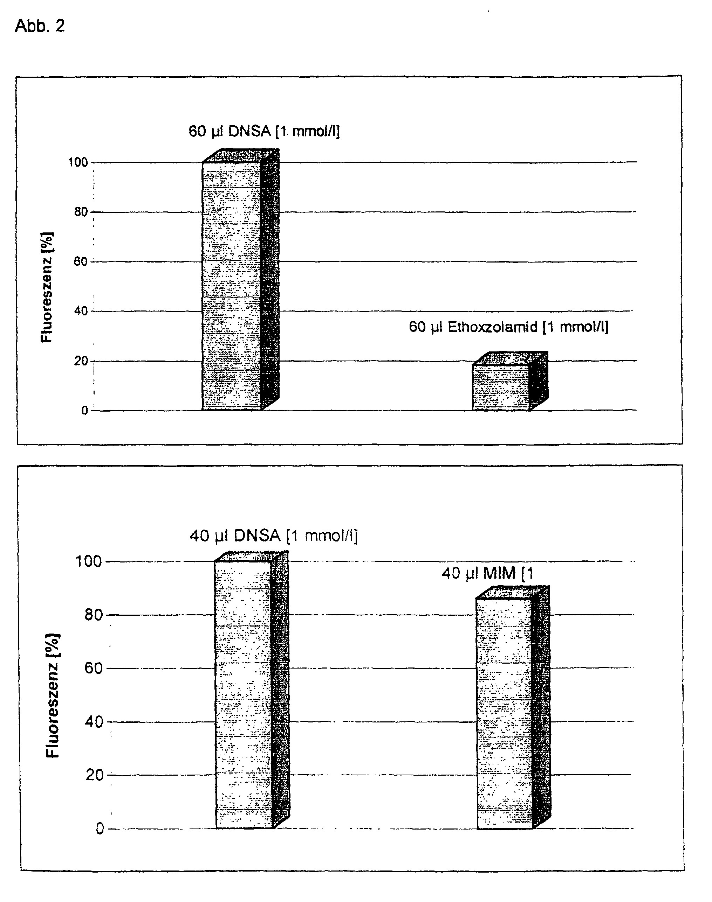

Der Vergleich mit Nierenzellen, die Carboanhydrase II besitzen, zeigt, daß 1 nicht aufgenommen wird, jedoch Ethoxzolamid (Abb. 2). Die Zellmembran von Erythrozyten ist dagegen teilweise für **1** durchlässig. Die Wirkung bleibt aber gering, da das Isoenzym II nur 15 % der erythrozytären Carbonbhydrase ausmacht.

### Wirkung auf die Knochenresorption:

### a) Zellkultur:

Behandelt man Osteoklasten, die auf Dentinplättchen angesiedelt wurden, mit **1**, so hören sie auf zu resorbieren; sie lösen sich von der Oberfläche und bleiben nur mehr durch einzelne Ausläufer mit ihr verbunden.

Auch in Gegenwart osteoklastenaktivierender Hormone findet keine Resorption mehr statt.

### b) in vivo (an Ratten):

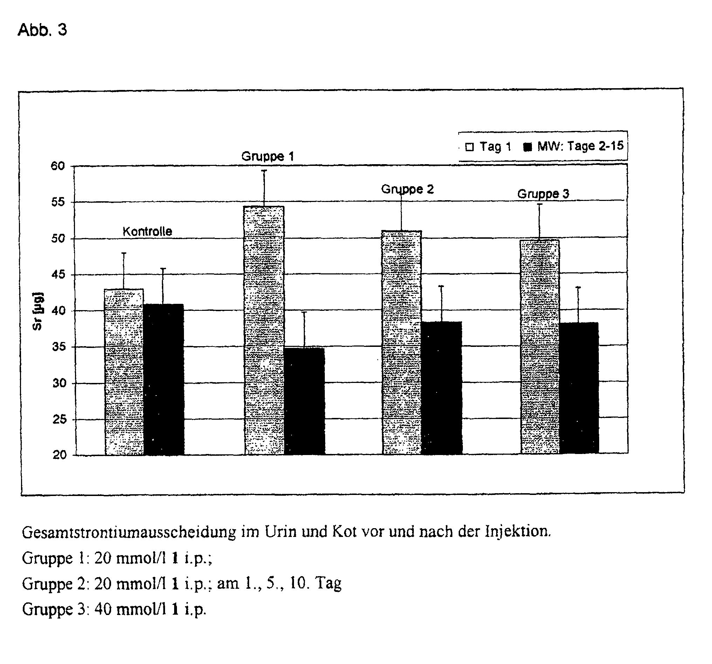

Wachsenden Ratten - 6 Wochen alt - wurden sechsmal in Abständen von drei Tagen jeweils 2 mg SrCl₂ in physiologischer Kochsalzlösung subkutan injiziert. Die Tiere bauen das Strontium analog dem Calcium in den Knochen ein. Entsprechend der Rate des Knochenumbaus wird das Strontium über Harn und Fäces während eines längeren Zeitraums wieder ausgeschieden. Nach einer 14 tägigen Latenzzeit in der sie eine calciumarme Diät erhielten, wurden ihnen verschiedene Konzentrationen von **1** i.p. apliziert und die Ausscheidung des Strontiums atomabsorptionsspektroskopisch gemessen. Die Abb. 3 zeigt die resultierende Erniedrigung der Strontiumausscheidung, was die verringerte Knochenresorption anzeigt.

Nach Abschluß des Versuches wurden die Tibiae präpariert und deren Gehalt an Calcium, Strontium und Phosphat bestimmt.

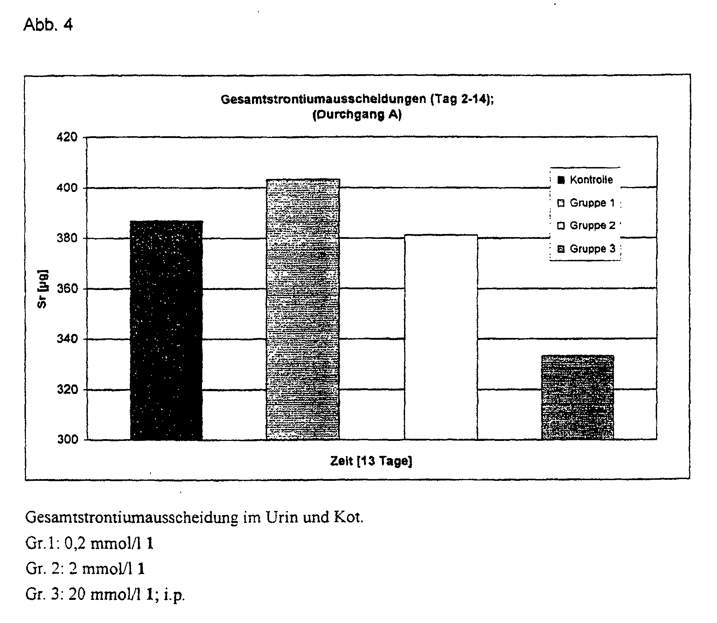

In allen Fällen ergab sich eine deutlich höhere Konzentration der genannten Ionen im Vergleich zu den Tieren der Kontrollgruppe, das heißt, auch die direkte Bestimmung dieser Knochenparameter ergibt, daß die Resorption gehemmt war (Abb. 4).

### Wirkung auf die Konzentration des Calciums im Serum:

Die Konzentration des Calciums im Serum wird in sehr engen Grenzen hormonell einreguliert und ist selbst unter pathologischen Bedingungen nur wenig verändert.

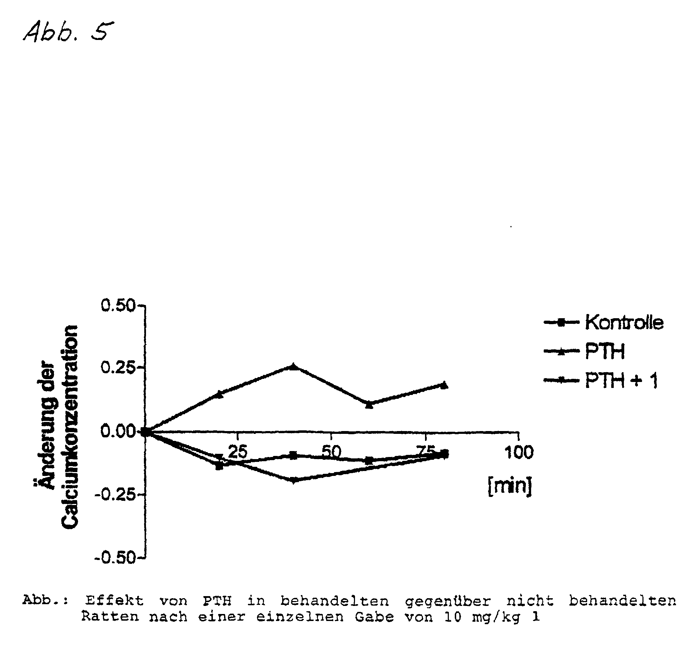

Die Hemmung der Knochenresorption in Ratten, die mit **1** behandelt wurden, kann an der Änderung der Calciumkonzentration im Serum nach Injektion von Parathormon demonstriert werden (Abb. 5). Selbst drei Tage nach einer einzelnen Dosis von 10 mg/kg **1** ist die knochenauflösende Wirkung von Parathormon vollständig blockiert.

### c) OVX-Ratten

Versuche an ovarektomierten Ratten über mehrere Monate belegen die Hemmwirkung von **1** auf die Knochenresooorption auch in diesem 'Osteoporose-Modell', wobei keine Nebenwirkungen zu beobachten waren.

## Patentansprüche

1. Mittel zur Behandlung von Knochenresorptionen, enthaltend einen Inhibitor, der säurebildende bzw. -transportierende Enzyme hemmt und der an Mannose oder Mannan gekoppelt vorliegt, wobei Mannose oder Mannan spezifisch an Rezeptoren der Osteoklasten binden.

2. Mittel nach Anspruch 1, **dadurch gekennzeichnet, dass** der Inhibitor Carboanhydrase hemmt.

3. Mittel nach Anspruch 1 oder 2, **dadurch gekennzeichnet, daß** der Inhibitor ein Sulfonamid ist.

4. Mittel nach Anspruch 3, **dadurch gekennzeichnet, daß** der Inhibitor ein Methazolamid, Azetazolamid, Ethoxzolamid, Chlorzolamid, p-Aminomethylbenzolsulfonamid (Marfanil), p-Aminobenzolsulfonamid (Sulfanilamid) oder 2,4-Diaminoazobenzol-4'-sulfonamid (Prontosil) ist.

5. Mittel nach einem der Ansprüche 1-4, **dadurch gekennzeichnet, daß** es aus N-α-D-Mannopyranosylphenyl-N'-p-amidosulfonylbenzylthioharnstoff besteht.

6. Mittel nach Anspruch 1, **dadurch gekennzeichnet, dass** der Inhibitor die H⁺-ATPase hemmt.

7. Mittel nach Anspruch 1 und 6, **dadurch gekennzeichnet, daß** der Inhibitor Omeprazol, Bafilomycin A1 oder Concanavalin ist

8. Verwendung des Mittels nach Anspruch 1-7 zur Herstellung eines Medikaments zur Behandlung von Knochenerkrankungen, die durch erhöhte Osteoklastenaktivität (erhöhte Resorption) bedingt sind.

9. Verwendung des Mittels nach Anspruch 1-7 zur Herstellung eines Medikaments zur Behandlung der Osteoporose.

## Claims

1. Means of treatment of bone resorptions, containing an inhibitor which inhibits acid-forming and transporting enzymes and which exists coupled to mannose or mannan, with mannose and mannan specifically binding to receptors of the osteoclasts.

2. Means according to Claim 1, wherein the inhibitor inhibits carboanhydrase.

3. Means according to Claim 1 or 2, wherein the inhibitor is a sulfonamide.

4. Means according to Claim 3, wherein the inhibitor is a methazolamide, acetazolamide, ethoxyzolamide, chlorzolamide, p-aminomethylbenzolsulfonamide (Marfanil), p-aminobenzolsulfonamide (sulfanilamide) or 2.4-diaminoazobenzol-4'-sulfonamide (Prontosil).

5. Means according to one of the Claims 1-4, wherein it comprises N-α-D-mannopyranoylphenyl-N'-p-amidosulfonylbenzylthio urea.

6. Means according to Claim 1, wherein the inhibitor inhibits the H⁺-ATPase.

7. Means according to Claims 1 and 6, wherein the inhibitor is Omeprazol, Bafilomycin Al or Concanavalin.

8. Use of the means according to Claims 1-7 for the production of a medication for the treatment of bone diseases caused by increased osteoclast activity (increased resorption).

9. Use of the means according to Claim 1-7 for the production of a medication for the treatment of osteoporosis.

## Revendications

1. Produit destiné au traitement des résorptions osseuses, contenant un inhibiteur qui inhibe les enzymes acidifiants resp. transporteurs d'acides et qui se trouve combiné à des mannoses ou mannanes, les mannoses ou mannanes liant spécifiquement aux récepteurs des ostéoblastes.

2. Produit selon la revendication 1, se caractérisant par le fait que l'inhibiteur carboanhydrase inhibe.

3. Produit selon la revendication 1 ou 2, se caractérisant par le fait que l'inhibiteur est un sulfamide.

4. Produit selon la revendication 3 se caractérisant par le fait que l'inhibiteur est un methazolamide, un azetazolamide, un ethoxzolamide, un chlorzolamide, un p-amino-methyl-benzol-sulfonamide (Marfanil), un p-amino-benzol-sulfonamide (sulfanilamide) ou 2.4-diaminoazobenzol-4'-sulfonamide (Prontosil).

5. Produit selon l'une des revendications 1 à 4, se caractérisant par le fait qu'il se compose de N-alpha-D-mannopyranosylphenyl-N'-p-aminosulfonylbenzylthio-urée.

6. Produit selon la revendication 1, se caractérisant par le fait que l'inhibiteur inhibe la H⁺-ATPase.

7. Produit selon la revendication 1 et 6, se caractérisant par le fait que l'inhibiteur est l'oméprazole, la bafilomycine A1 ou la concanavaline.

8. Emploi du produit selon la revendication 1 à 7 pour la fabrication d'un médicament destiné au traitement de maladies osseuses qui sont conditionnées par une augmentation de l'activité des ostéoclastes (augmentation de la résorption).

9. Emploi du produit selon la revendication 1 à 7 pour la fabrication d'un médicament destiné au traitement de l'ostéoporose.
